# EUROPÄISCHE PATENTSCHRIFT

(11) **EP 3 389 730 B1**
(45) Veröffentlichungstag und Bekanntmachung des Hinweises auf die Patenterteilung: **22.01.2025**
(21) Anmeldenummer: 16802023.8
(22) Anmeldetag: 24.11.2016
(51) Int. Cl.: A61L 2/14, A61L 2/20, C23C 16/04, B65D 23/02

(54) **VERFAHREN UND VORRICHTUNG ZUR PLASMABEHANDLUNG VON BEHÄLTERN**
METHOD AND DEVICE FOR THE PLASMA PROCESSING OF CONTAINERS
PROCÉDÉ ET DISPOSITIF DE TRAITEMENT PAR PLASMA DE RÉCIPIENTS

(30) Priorität: 14.12.2015 DE 102015121773
(43) Veröffentlichungstag der Anmeldung: 24.10.2018
(73) Patentinhaber: KHS GmbH, 44143 Dortmund (DE)
(72) Erfinder: VORWERK, Jürgen, Franz, 56290 Mörsdorf (DE); SINGUR, Igor, 55545 Bad Kreuznach (DE); KYTZIA, Sebastian, 23826 Todesfelde (DE)
(74) Vertreter: Eisenführ Speiser
(86) Internationale Anmeldenummer: PCT/EP2016/078731
(87) Internationale Veröffentlichungsnummer: WO 2017/102280

(56) Entgegenhaltungen:
- EP-A1- 2 151 510
- EP-A1- 2 772 447
- EP-A1- 3 199 489
- WO-A1-00/00394
- WO-A1-2011/153993
- WO-A2-2008/083824
- DE-A1- 10 224 395
- DE-A1- 10 225 609
- DE-A1- 102008 016 923
- DE-A1- 19 944 631
- JP-A- 2005 081 309
- US-A1- 2001 042 510
- US-A1- 2006 150 909
- US-A1- 2009 304 950

## Beschreibung

Die Erfindung bezieht sich auf ein Verfahren zur Plasmabehandlung von Behältern gemäß dem Oberbegriff des Patentanspruches 1. Die Erfindung betrifft weiterhin eine Vorrichtung zur Plasmabehandlung von Behältern gemäß dem Oberbegriff des Patentanspruches 10. Derartige Verfahren und Vorrichtungen werden beispielsweise eingesetzt, um Kunststoffe mit Oberflächenbeschichtungen zu versehen. Insbesondere sind auch bereits derartige Vorrichtungen bekannt, um innere oder äußere Oberflächen von Behältern zu beschichten, die zur Verpackung von Flüssigkeiten vorgesehen sind. Darüber hinaus sind Einrichtungen zur Plasmasterilisation bekannt.

DE19944631 offenbart eine Vorrichtung zur Entkeimung mittels Wasserstoffperoxid und Beschichtung von Behältern durch Mikrowellenplasma.

In der Druckschrift WO95/22413 A1 wird eine Plasmakammer zur Innenbeschichtung von Behältern aus PET beschrieben. Die zu beschichtenden Behälter werden durch einen beweglichen Boden in eine Plasmakammer hineingehoben und im Bereich einer Behältermündung mit einem Adapter in Verbindung gebracht. Durch den Adapter hindurch kann eine Evakuierung des Behälterinnenraumes erfolgen. Darüber hinaus wird durch den Adapter hindurch eine hohle Gaslanze in den Innenraum der Behälter eingeführt, um Prozessgas zuzuführen. Eine Zündung des Plasmas erfolgt unter Verwendung einer Mikrowelle.

Aus dieser Veröffentlichung ist es auch bereits bekannt, eine Mehrzahl von Plasmakammern auf einem rotierenden Rad anzuordnen. Hierdurch wird eine hohe Produktionsrate von Behältern je Zeiteinheit unterstützt.

In der Druckschrift EP 10 10 773 A1 wird eine Zuführeinrichtung erläutert, um einen Flascheninnenraum zu evakuieren und mit Prozessgas zu versorgen. In der WO 01/31680 A1 wird eine Plasmakammer beschrieben, in die die Flaschen von einem beweglichen Deckel eingeführt werden, der zuvor mit einem Mündungsbereich der Flaschen verbunden wurde.

Die Druckschrift WO 00/58631 A1 zeigt ebenfalls bereits die Anordnung von Plasmastationen auf einem rotierenden Rad und beschreibt für eine derartige Anordnung eine gruppenweise Zuordnung von Unterdruckpumpen und Plasmastationen, um eine günstige Evakuierung der Kammern sowie der Innenräume der Flaschen zu unterstützen. Darüber hinaus wird die Beschichtung von mehreren Behältern in einer gemeinsamen Plasmastation bzw. einer gemeinsamen Kavität erwähnt.

Eine weitere Anordnung zur Durchführung einer Innenbeschichtung von Flaschen wird in der Druckschrift WO 99/17334 A1 beschrieben. Es wird hier insbesondere eine Anordnung eines Mikrowellengenerators oberhalb der Plasmakammer sowie eine Vakuum- und Betriebsmittelzuleitung durch einen Boden der Plasmakammer hindurch beschrieben.

In der DE 10 2004 020 185 A1 wird bereits eine Gaslanze beschrieben, die in den Innenraum eines zu beschichtenden Vorformlings einfahrbar ist und zur Zuleitung von Prozessgasen dient. Die Gaslanze ist in der Längsrichtung des Behälters positionierbar.

Stand der Technik zum Gegenstand der Erfindung ist weiterhin beispielsweise zu finden in der EP 2 151 510 A1, US 2001/042510 A1, EP 2 772 447 A1 und WO 00/00394 A1.

Bei der überwiegenden Anzahl der bekannten Vorrichtungen werden zur Verbesserung von Barriereeigenschaften des thermoplastischen Kunststoffmaterials durch das Plasma erzeugte Behälterschichten aus Siliziumoxiden mit der allgemeinen chemischen Formel SiOx verwendet. Derartige Barriereschichten insbesondere an der Innenwandung eines Behälterinnenraums verhindern ein Eindringen von Sauerstoff in die verpackten Flüssigkeiten sowie ein Austreten von Kohlendioxid bei CO2-haltigen Flüssigkeiten und verbessern damit die Lagerungseigenschaften bzw. die dauerhafte Haltbarkeitsmachung der in dem Behälter abgefüllten und/oder verpackten Flüssigkeiten.

Aufgabe der Erfindung ist es, ein Verfahren und eine Vorrichtung zur Plasmabehandlung von Behältern anzugeben, bei dem die Lagerungseigenschaften des Behälterinnenraums im Vergleich zum Stand der Technik weitergehend verbessert werden. Zur Lösung dieser Aufgabe ist ein Verfahren zur Plasmabehandlung von Werkstücken entsprechend dem Patentanspruch 1 ausgebildet. Eine Vorrichtung zur Plasmabehandlung von Behältern bildet den Gegenstand des Patentanspruches 10.

Der wesentliche Aspekt gemäß des erfindungsgemäßen Verfahrens besteht darin, dass zumindest der Behälterinnenraum des wenigstens einen Behälters nach der Plasmabehandlung und vor dem Befüllen des Behälters in der Plasmakammer mit einem gas-, dampf- oder nebelförmigen Sterilisationsmedium beaufschlagt wird und zwar bevorzugt derart, dass bei dem Belüftungsschritt zumindest der Behälterinnenraum zumindest teilweise mit dem gas-, dampf- oder nebelförmigen Sterilisationsmedium belüftet wird. Dabei bezieht sich der Aggregatszustand des vorgenannten Sterilisationsmediums auf Atmosphärendruck oder auf den Aggregatszustand des Fluids in der entsprechenden Zuleitung.

Besonders vorteilhaft wird damit unterschiedlich zum Stand der Technik die evakuierte Plasmakammer einschließlich des darin befindlichen Behälters nicht wie bisher im Stand der Technik üblich mit Umgebungsdruck belüftet, sondern gezielt über deren Belüftung ein Sterilisationsmedium eingeleitet, um damit eine sterilisierende Atmosphäre innerhalb des Behälterinnenraumes für den späteren Füllvorgang bereits am Ende der Plasmabehandlung, und damit insbesondere noch an dem Plasmamodul, geschaffen. Erfindungsgemäß eignet sich dabei als Sterilisationsmedium ein Wasserstoffperoxyd (H2O2) enthaltendes Sterilisationsmedium, das beispielweise zusammen mit heißer steriler Luft verwendet wird und sich damit an dem kälteren Behälterinnenraum als H2O2-Kondensationsfilm niederschlägt. Erfindungsgemäß ist auch ein alternativ oder zusätzlich Ozon enthaltendes Sterilisationsmedium.

Besonders vorteilhaft kann die Belüftung des Behälterinnenraumes mit Sterilisationsmedium derart erfolgt, dass beim Schritt des Belüftens mindestens einmal ein Sterilisationsmedium in den Behälterinnenraum eingeleitet wird. Damit kann besonders vorteilhaft über den Belüftungsschritt das Sterilisationsmedium in zumindest den Behälterinnenraum des Behälters eingeleitet werden. Dabei soll unter Belüften oder dem Schritt des Belüftens das Einleiten eines Fluides verstanden werden, insb. umfassend oder bestehend aus einem Gas oder Gasgemisch zum Zwecke des Anhebens des Vakuums bzw. des Unterdruckes in einer evakuierten Kammer und/oder in einem Behälterinnenraum.

Vorteilhafterweise kann die Belüftung mittels des Sterilisationsmediums an der Plasmastation eines Plasmarades erfolgen, wobei mindestens eine nachfolgende Behandlung im Bereich eines Ausgaberades oder der Überleitstrecke zur nächsten Behandlungsmaschine erfolgen kann. Weiterhin vorteilhaft kann der Behälterinnenraum des Behälters wenigstens bis auf Atmosphärendruck oder darüber hinaus, also mit Überdruck, mit dem Sterilisationsmedium beaufschlagt werden. Bevorzugt kann dabei vorgesehen sein, dass die Beaufschlagung wenigstens des Behälterinnenraums des Behälters mit einem Wasserstoffperoxid und/oder Ozon enthaltenden Sterilisationsmedium erfolgt.

In einer vorteilhaften Ausführungsvariante kann neben dem Behälterinnenraum des Behälters auch die Plasmakammer und damit auch die Behälteraußenfläche wenigstens teilweise mit dem Sterilisationsmedium belüftet werden.

Besonders bevorzugt kann auch vorgesehen sein, dass eine nachfolgende Aktivierung und/oder Trocknung des Sterilisationsmediums erfolgt. Dafür geeignete Aktivierungs- und Trocknungseinrichtungen sind dem Fachmann einschlägig bekannt. Vorteilhafterweise kann zumindest die Aktivierung des Sterilisationsmediums für den jeweiligen Behälter im Bereich des Ausgaberades erfolgen. Alternativ vorteilhaft kann die Aktivierung des jeweiligen Behälters auch während seines Transports zu einer einem Plasmamodul nachgelagerten Behälterbehandlungsmaschine erfolgen. In einer bevorzugten Ausführungsvariante kann auch vorgesehen sein, dass die Trocknung des jeweiligen Behälters während seines Transports zu einer einem Plasmamodul nachgelagerten Behälterbehandlungsmaschine erfolgt.

In einer bevorzugten Ausführungsvariante kann vor einer erneuten Plasmabehandlung wenigstens eine erste bis dritte Prozessgasleitung sowie eine zentrale Prozessgasleitung abgesaugt und/oder gespült werden.

Vorteilhaft kann die Belüftung mittels Sterilisationsmedium wenigstens über eine in die zentrale Prozessgasleitung fluiddicht einmündende zweite Belüftungsleitung erfolgen, die über eine Ventileinrichtung gesteuert und/oder geregelt zu- und/oder abschaltbar ausgebildet ist.

Vorteilhaft kann die Belüftung mittels Sterilisationsmedium auch über eine in eine zweite Seite eines Vakuumkanals fluiddicht einmündende weitere erste Belüftungsleitung erfolgen, die über eine Ventileinrichtung (76.1) gesteuert und/oder geregelt zu- und/oder abschaltbar ausgebildet ist.

Wiederum vorteilhaft kann die Belüftung mittels Sterilisationsmedium über eine nochmals weitere dritte fluiddicht in die Plasmakammer einmündende Belüftungsleitung erfolgen, die über eine Ventileinrichtung gesteuert und/oder geregelt zu- und/oder abschaltbar ausgebildet ist.

In einer vorteilhaften Ausgestaltung wird in einer Fluidverbindung zwischen der zentralen Vakuumvorrichtung eine zusätzliche Absperrventileinrichtung, idealerweise ein Dreiwegeventil vorgesehen. Diese Absperreinrichtung ermöglicht, dass mindestens zeitweise während des Belüftens mit einem Sterilisationsmittel eine räumliche Abgrenzung der Leitungen vorgenommen werden kann, in dem diese Absperrvorrichtung bedarfsabhängig, zeitweise geschlossen wird. Somit wird eine Kontamination mindestens einer der Vakuumleitungen, einer Gruppe von Vakuumleitungen oder von Teilabschnitten einer oder mehrerer Vakuumleitungen mit Sterilisationsmedium sicher verhindert oder soweit wie möglich eingeschränkt.

Unter "Behälter" werden im Rahmen der Erfindung insbesondere Dosen, Flaschen, Fässer, auch KEGs, Tuben, Pouches, jeweils aus Metall, Glas und/oder Kunststoff, verstanden, aber auch andere Packmittel, insbesondere auch solche die zum Abfüllen von pulverförmigen, granulatartigen, flüssigen oder viskosen Produkten geeignet sind.

Der Ausdruck "im Wesentlichen" bzw. "etwa" bzw. "ca." bedeutet im Sinne der Erfindung Abweichungen vom jeweils exakten Wert um +/- 10%, bevorzugt um +/- 5% und/oder Abweichungen in Form von für die Funktion unbedeutenden Änderungen.

Weiterbildungen, Vorteile und Anwendungsmöglichkeiten der Erfindung ergeben sich auch aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Die Erfindung wird im Folgenden anhand der Figuren an Ausführungsbeispielen näher erläutert. Es zeigen:
- Fig. 1: eine Prinzipskizze einer Mehrzahl von Plasmakammern, die auf einem rotierenden Plasmarad angeordnet sind und bei der das Plasmarad mit Eingabe- und Ausgaberädern gekoppelt ist,
- Fig. 2: eine Anordnung gemäß Fig. 1, bei der die Plasmastationen jeweils zwei Plasmakammern aufweisen,
- Fig. 3: eine perspektivische Darstellung eines Plasmarades mit einer Vielzahl von Plasmakammern,
- Fig. 4: eine perspektivische Darstellung einer Plasmastation mit einer Kavität,
- Fig. 5: eine Vorderansicht der Vorrichtung gemäß Fig. 4 mit geschlossener Plasmakammer,
- Fig. 6: einen Querschnitt gemäß Schnittlinie VI-VI in Fig. 5,
- Fig. 7: ein schematisches Blockschaltbild einer erfindungsgemäß ausgebildeten Plasmastation.

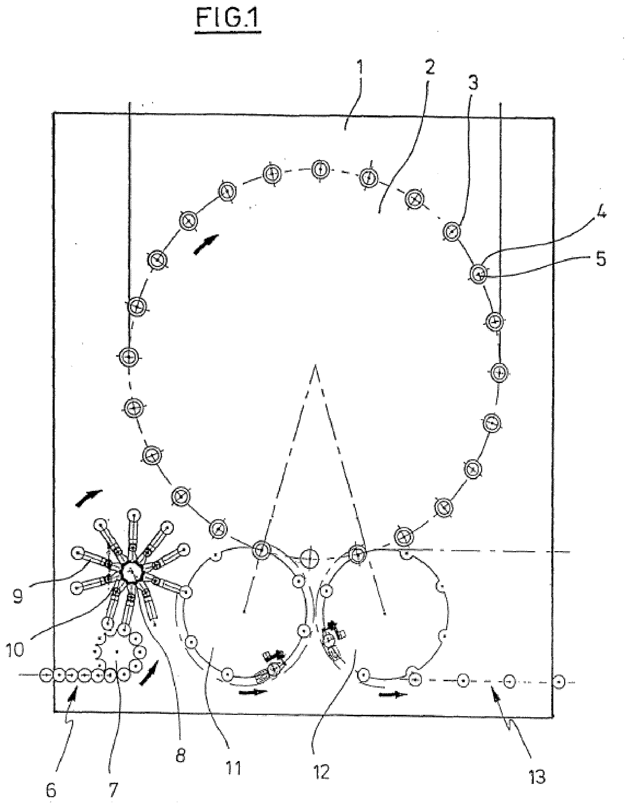

In Figur 1 ist mit dem Bezugszeichen 1 allgemein ein Plasmamodul bezeichnet, das mit einem rotierenden Plasmarad 2 versehen ist. Entlang eines Umfanges des Plasmarades 2 befinden sich eine Mehrzahl von Plasmastationen 3 angeordnet. Die Plasmastationen 3 sind mit Kavitäten 4 bzw. Plasmakammern 17 zur Aufnahme von zu behandelnden Behältern 5 mit jeweils zumindest einem Behälterinnenraum 5.1 versehen.

Die zu behandelnden Behälter 5 werden dem Plasmamodul 1 im Bereich einer Eingabe 6 zugeführt und über ein Vereinzelungsrad 7 an ein Übergaberad 8 weitergeleitet, das mit positionierbaren Tragarmen 9 ausgestattet ist. Die Tragarme 9 sind relativ zu einem Sockel 10 des Übergaberades 8 verschwenkbar angeordnet, so dass eine Abstandsveränderung der Behälter 5 relativ zueinander durchgeführt werden kann. Hierdurch erfolgt eine Übergabe der Behälter 5 vom Übergaberad 8 an ein Eingaberad 11 mit einem relativ zum Vereinzelungsrad 7 vergrößerten Abstand der Behälter 5 relativ zueinander. Das Eingaberad 11 übergibt die zu behandelnden Behälter 5 an das Plasmarad 2. Nach einer Durchführung der Behandlung werden die behandelten Behälter 5 von einem Ausgaberad 12 aus dem Bereich des Plasmarades 2 entfernt und in den Bereich einer Ausgabestrecke 13 überführt.

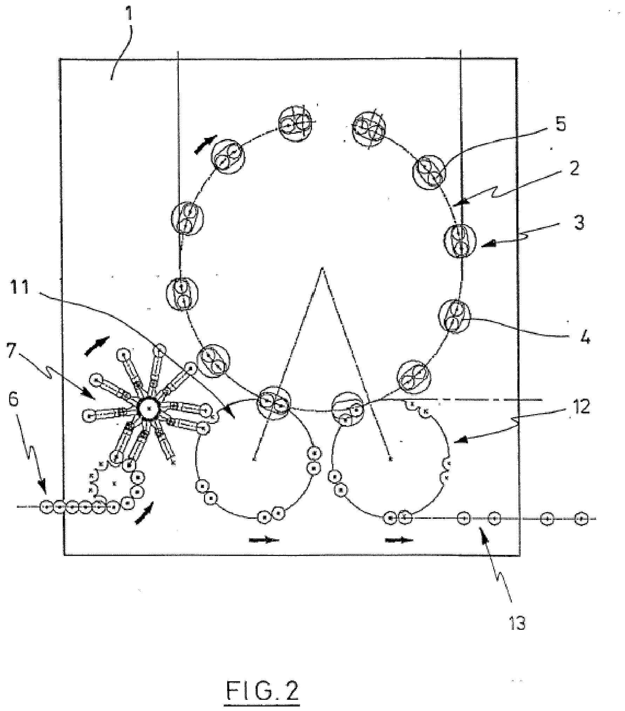

Bei der Ausführungsform gemäß Figur 2 sind die Plasmastationen 3 jeweils mit zwei Kavitäten 4 bzw. Plasmakammern 17 ausgestattet. Hierdurch können jeweils zwei Behälter 5 gleichzeitig behandelt werden. Grundsätzlich ist es hierbei möglich, die Kavitäten 4 vollständig voneinander getrennt auszubilden, grundsätzlich ist es aber auch möglich, in einem gemeinsamen Kavitätenraum lediglich Teilbereiche derart abzugrenzen, dass eine optimale Beschichtung aller Behälter 5 gewährleistet ist. Insbesondere ist hierbei daran gedacht, die Teilkavitäten zumindest durch separate Mikrowelleneinkopplungen gegeneinander abzugrenzen.

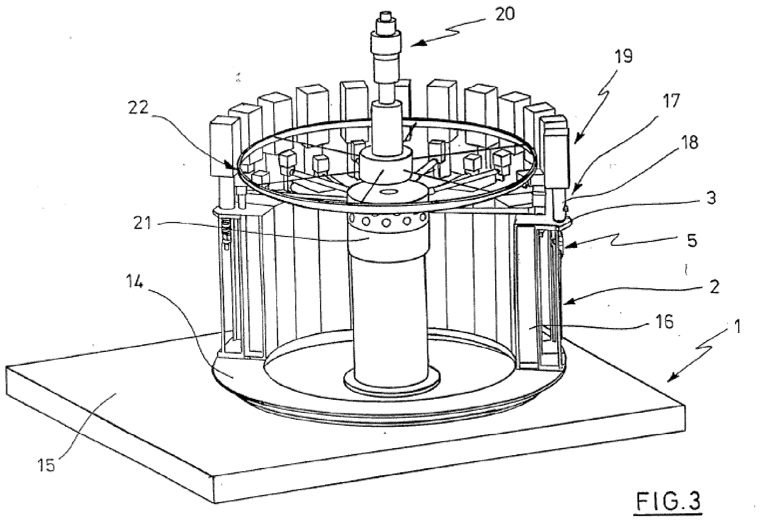

Figur 3 zeigt eine perspektivische Darstellung eines Plasmamoduls 1 mit teilweise aufgebautem Plasmarad 2. Die Plasmastationen 3 sind auf einem Tragring 14 angeordnet, der als Teil einer Drehverbindung ausgebildet und im Bereich eines Maschinensockels 15 gelagert ist. Die Plasmastationen 3 weisen jeweils einen Stationsrahmen 16 auf, der die Plasmakammern 17 haltert. Die Plasmakammern 17 können zylinderförmige Kammerwandungen 18 sowie Mikrowellengeneratoren 19 aufweisen.

In einem Zentrum des Plasmarades 2 kann ein Drehverteiler 20 vorgesehen sein, über den die Plasmastationen 3 mit Betriebsmitteln sowie Energie versorgt werden. Zur Betriebsmittelverteilung können insbesondere Ringleitungen 21 eingesetzt werden.

Die zu behandelnden Behälter 5 sind unterhalb der zylinderförmigen Kammerwandungen 18 dargestellt, wobei Unterteile der Plasmakammern 17 zur Vereinfachung jeweils nicht eingezeichnet sind.

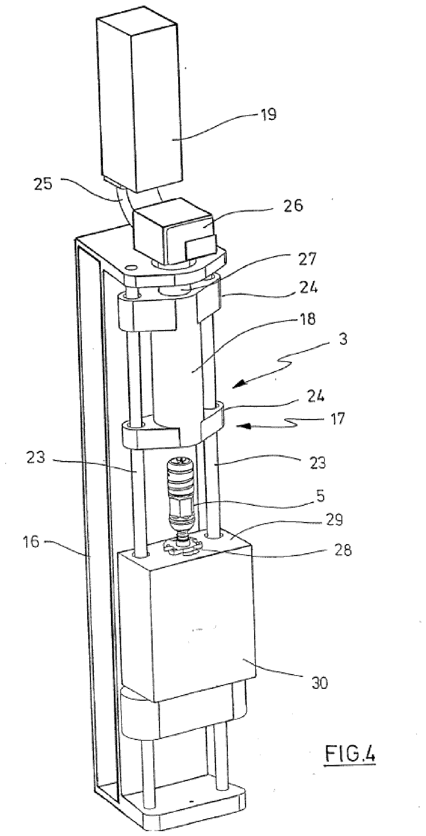

Figur 4 zeigt eine Plasmastation 3 in perspektivischer Darstellung. Es ist zu erkennen, dass der Stationsrahmen 16 mit Führungsstangen 23 versehen ist, auf denen ein Schlitten 24 zur Halterung der zylinderförmigen Kammerwandung 18 geführt ist. Figur 4 zeigt den Schlitten 24 mit Kammerwandung 18 in einem angehobenen Zustand, so dass der Behälter 5 freigegeben ist.

Im oberen Bereich der Plasmastation 3 ist der Mikrowellengenerator 19 angeordnet. Der Mikrowellengenerator 19 ist über eine Umlenkung 25 und einen Adapter 26 an einen Kopplungskanal 27 angeschlossen, der in die Plasmakammer 17 einmündet. Grundsätzlich kann der Mikrowellengenerator 19 sowohl unmittelbar im Bereich des Kammerdeckels 31 als auch über ein Distanzelement an den Kammerdeckel 31 angekoppelt mit einer vorgebbaren Entfernung zum Kammerdeckel 31 und somit in einem größeren Umgebungsbereich des Kammerdeckels 31 angeordnet werden. Der Adapter 26 hat die Funktion eines Übergangselementes und der Kopplungskanal 27 ist als ein Koaxialleiter ausgebildet. Im Bereich einer Einmündung des Kopplungskanals 27 in den Kammerdeckel 31 ist ein Quarzglasfenster angeordnet. Die Umlenkung 25 ist als ein Hohlleiter ausgebildet.

Der Behälter 5 wird von einem Halteelement 28 positioniert, das im Bereich eines Kammerbodens 29 angeordnet ist. Der Kammerboden 29 ist als Teil eines Kammersockels 30 ausgebildet. Zur Erleichterung einer Justage ist es möglich, den Kammersockel 30 im Bereich der Führungsstangen 23 zu fixieren. Eine andere Variante besteht darin, den Kammersockel 30 direkt am Stationsrahmen 16 zu befestigen. Bei einer derartigen Anordnung ist es beispielsweise auch möglich, die Führungsstangen 23 in vertikaler Richtung zweiteilig auszuführen.

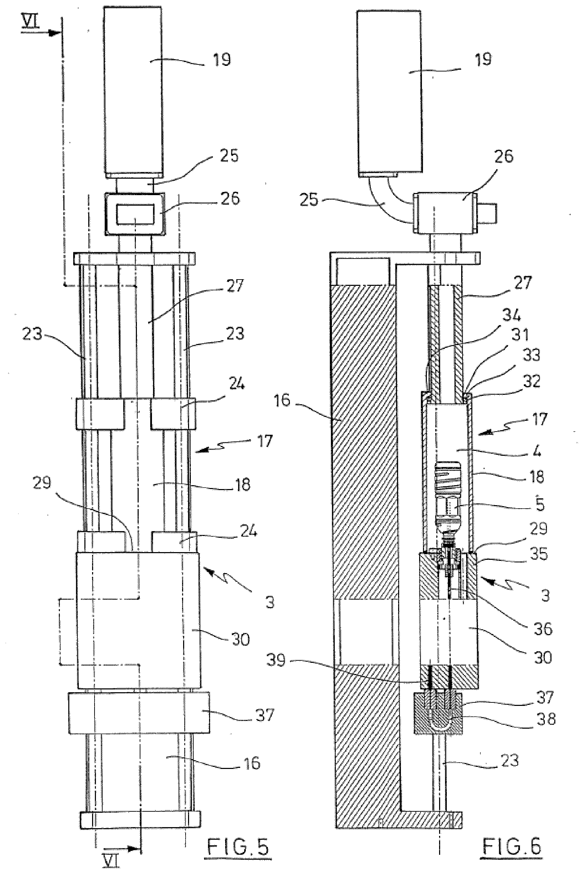

Figur 5 zeigt eine Vorderansicht der Plasmastation 3 gemäß Figur 3 in einem geschlossenen Zustand der Plasmakammer 17. Der Schlitten 24 mit der zylinderförmigen Kammerwandung 18 ist hierbei gegenüber der Positionierung in Figur 4 abgesenkt, so dass die Kammerwandung 18 gegen den Kammerboden 29 gefahren ist. In diesem Positionierzustand kann die Plasmabeschichtung durchgeführt werden.

Figur 6 zeigt in einer Vertikalschnittdarstellung die Anordnung gemäß Figur 5. Es ist insbesondere zu erkennen, dass der Kopplungskanal 27 in einen Kammerdeckel 31 einmündet, der einen seitlich überstehenden Flansch 32 aufweist. Im Bereich des Flansches 32 ist eine Dichtung 33 angeordnet, die von einem Innenflansch 34 der Kammerwandung 18 beaufschlagt wird. In einem abgesenkten Zustand der Kammerwandung 18 erfolgt hierdurch eine Abdichtung der Kammerwandung 18 relativ zum Kammerdeckel 31. Eine weitere Dichtung 35 ist in einem unteren Bereich der Kammerwandung 18 angeordnet, um auch hier eine Abdichtung relativ zum Kammerboden 29 zu gewährleisten.

In der in Figur 6 dargestellten Positionierung umschließt die Kammerwandung 18 die Kavität 4, so dass sowohl ein Innenraum der Kavität 4 als auch ein Behälterinnenraum 5.1 des Behälters 5 evakuiert werden können. Zur Unterstützung einer Zuleitung von Prozessgas ist im Bereich des Kammersockels 30 eine hohle Gaslanze 36 angeordnet, die in den Behälterinnenraum 5.1 des Behälters 5 hineinverfahrbar ist. Zur Durchführung einer Positionierung der Gaslanze 36 wird diese von einem Lanzenschlitten 37 gehaltert, der entlang der Führungsstangen 23 positionierbar ist. Innerhalb des Lanzenschlittens 37 verläuft ein Prozessgaskanal 38, der in der in Figur 6 dargestellten angehobenen Positionierung mit einem Gasanschluss 39 des Kammersockels 30 gekoppelt ist. Durch diese Anordnung werden schlauchartige Verbindungselemente am Lanzenschlitten 37 vermieden. Im in den Behälterinnenraum 5.1 eingefahrenen Zustand der Gaslanze 36 ist der Behälterinnenraum 5.1 des Behälters 5 gegenüber dem Innenraum der Kavität 4 isoliert, d. h. abgedichtet. Wohingegen in einem abgesenkten Zustand der Gaslanze 36 eine gasdurchlässige Verbindung zwischen dem Behälterinnenraum 5.1 des Behälters 5 und dem Innenraum der Kavität 4 geschaffen wird.

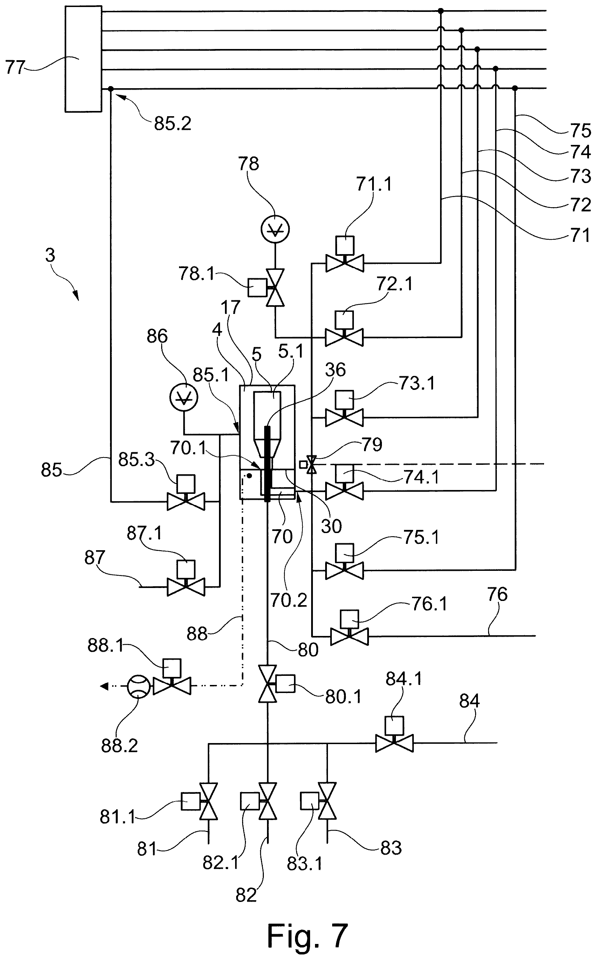

Figur 7 zeigt beispielhaft ein schematisches Blockschaltbild exemplarisch an einer Plasmastation 3 des Plasmamoduls 1. Das Plasmamodul 3 umfasst dabei wenigstens die Plasmakammer 17, in deren Innenraum der Kavität 4 der Behälter 5 gas- und/oder luftdicht eingesetzt und positioniert wird, sowie den Kammersockel 30, der wenigstens einen Vakuumkanal 70 aufweist. Der Vakuumkanal 70 mündet dabei mit seiner ersten Seite 70.1 in der Plasmakammer 17 bzw. stellt je nach Stellung der Gaslanze 36 zusätzlich auch eine gasdurchlässige Verbindung in den Behälterinnenraum 5.1 des Behälters 5 her. Insbesondere kann vorgesehen sein, dass in einem in den Behälterinnenraum 5.1 eingefahrenen Zustand der Gaslanze 36 der Behälterinnenraum 5.1 gegenüber dem Innenraum der Kavität 4 isoliert, d. h. abgedichtet, ist, wohingegen in einem abgesenkten Zustand der Gaslanze 36 eine gasdurchlässige Verbindung zwischen dem Behälterinnenraum 5.1 des Behälters 5 und dem Innenraum der Kavität 4 geschaffen wird.

Ferner kann an einer zweiten Seite 70.2 des Vakuumkanals 70 wenigstens eine erste bis fünfte Vakuumleitung 71 ... 75 sowie wenigstens eine erste Belüftungsleitung 76 für ein Sterilisationsmedium angeschlossen sein, wobei insbesondere die erste Belüftungsleitung 76 über eine regel- und/oder steuerbare Ventileinrichtung 76.1 zu- bzw. abschaltbar ausgebildet ist. Zudem kann auch eine jede der ersten bis fünften Vakuumleitung 71...75 jeweils zumindest eine regel- und/oder steuerbare Ventileinrichtung 71.1 ...75.5 umfassen, wobei die Ventileinrichtungen 71.1...76.1 über eine nicht näher dargestellte Maschinensteuerung des Plasmamoduls 1 ansteuerbar ausgebildet sind.

An dem der zweiten Seite 70.2 des Vakuumkanals 70 abgewandten Ende stehen die erste bis fünfte Vakuumleitung 71...75 vorzugsweise mit einer für alle Vakuumleitungen 71 ...75 gemeinsamen Vakuumeinrichtung 77 in fluiddichter Verbindung. Die Vakuumeinrichtung 77 ist dabei insbesondere zur Erzeugung des in der Plasmakammer 17 sowie des Behälterinnenraums 5.1 während der Plasmabehandlung notwendigen Vakuums eingerichtet. Weiterhin ist Vakuumeinrichtung 77 dazu eingerichtet, an der ersten bis fünften Vakuumleitung 71 ...75 unterschiedliche Unterdrücke, also Unterdruckstufen je Vakuumleitung 71 ...75, zu erzeugen. Alternativ ist es jedoch auch möglich, die einzelnen Vakuumleitungen 71 ...75 an jeweils separate Vakuumeinrichtungen 77 anzuschließen.

Insbesondere kann vorgesehen sein, dass über die erste bis fünfte Vakuumleitung 71 ...75 die Plasmakammer 17 und/oder der Behälterinnenraum 5.1 auf unterschiedliche Unterdruckstufen abgesenkt werden. Beispielsweise kann hierfür angedacht sein, dass über die erste Vakuumleitung 71 bei geöffneter Ventileinrichtung 71.1 die Plasmakammer 17 einschließlich des Behälterinnenraums 5.1 auf eine erste Unterdruckstufe abgesenkt werden, während beispielsweise bei Öffnen der Ventileinrichtung 72.1 der zweiten Vakuumleitung 72 eine gegenüber der ersten Unterdruckstufe niedrigere Unterdruckstufe sowohl in der Plasmakammer 17 als auch in dem Behälterinnenraum 5.1 geschaffen wird. Weiterhin kann auch vorgesehen sein, dass beispielweise die fünfte Vakuumleitung 75 als Prozessvakuumleitung ausgebildet ist, die zur Aufrechterhaltung des Vakuums synchron zur Zuführung eines Prozessgases während der Plasmabehandlung eingerichtet ist. Damit vermeidet die vorgesehene Prozessvakuumleitung einen Übertritt von abgesaugtem Prozessgas in die Versorgungskreise der weiteren Vakuumleitungen, beispielweise der ersten bis vierten Vakuumleitung 71 ...74.

Auch kann der ersten bis fünften Vakuumleitung 71...75 eine beispielweise als Druckmessröhre ausgebildete Druckmesseinrichtung 78 zugeordnet sein, die dazu eingerichtet ist, den über die erste bis fünfte Vakuumleitung 71 ...75 erzeugten Unterdruck zu erfassen. Insbesondere kann der Druckmesseinrichtung 78 eine vorgeschaltete Ventileinrichtung 78.1 zugeordnet sein und die Druckmesseinrichtung 78 in einer Fluidverbindung der zweiten Vakuumleitung 72 zur zweiten Seite 70.2 des Vakuumkanals 70 angeordnet werden.

Weiterhin kann vorzugsweise in einer Fluidverbindung zwischen der dritten Vakuumleitung 73 und der vierten Vakuumleitung 74 mit jeweils der zweiten Seite 70.2 des Vakuumkanals 70 eine zusätzliche Absperrventileinrichtung 79 vorgesehen sein, die ebenfalls regel- und/oder steuerbar ausgebildet ist und die insbesondere während des Belüftens zumindest des Behälterinnenraumes 5.1 mit dem Sterilisationsmedium geschlossen wird, um eine Kontamination der Versorgungskreise beispielweise wenigstens der ersten bis dritten Vakuumleitung 71 ...73 mit Sterilisationsmedium zu verhindern, d. h. abzusperren. Weiterhin kann auch vorgesehen sein, dass über die Absperrventileinrichtung 79 nach erfolgtem Belüften zumindest des Behälterinnenraums 5.1 ein Spülmedium in die Versorgungskreise eingeleitet wird, und dadurch die mit Sterilisationsmedium kontaminierten Versorgungskreise gereinigt werden.

Zudem kann die Gaslanze 36 über eine beispielweise zentrale Prozessgasleitung 80 mit beispielweise einer ersten bis dritten Prozessgasleitung 81 ...83 gekoppelt sein, über die jeweils unterschiedliche Prozessgaszusammensetzungen insbesondere dem Behälterinnenraum 5.1 mittels der Gaslanze 36 zuführbar sind. Eine jede der ersten bis dritten Prozessgasleitungen 81...83 kann dabei ferner jeweils zumindest eine beispielweise über die zentrale Maschinensteuerung des Plasmamoduls 1 regel- und/oder steuerbare Ventileinrichtung 81.1...83.1 aufweisen. Mithin kann auch die zentrale Prozessgasleitung 80 eine derartige Steuer- und/oder regelbare Ventileinrichtung 80.1 umfassen. Ferner kann an der beispielweise zentralen Prozessgasleitung 80 auch eine zweite Belüftungsleitung 84 für das Sterilisationsmedium angeschlossen sein, die über eine regel- und/oder steuerbare Ventileinrichtung 84.1 zu- bzw. abschaltbar ausgebildet ist und mittels der zumindest der Behälterinnenraum 5.1 mit dem Sterilisationsmedium beaufschlagbar, also in diese einleitbar, ist. Beispielweise kann die zweite Belüftungsleitung 84 in eine Fluidverbindung der dritten Prozessgasleitung 83 in die zentrale Prozessgasleitung 80 fluiddicht einmünden.

Weiterhin kann auch eine sechste Vakuumleitung 85 mit einer ersten Seite 85.1 unmittelbar und insbesondere fluiddicht mit der Plasmakammer 17 verbunden sein bzw. in diese einmünden, und mit einer zweiten Seite 85.2 unter Zwischenschaltung einer regel- und/oder steuerbaren Ventileinrichtung 85.3 über die fünfte Vakuumleitung 75 mit der zentralen Vakuumeinrichtung 77 fluiddicht zusammenwirken. Auch kann der sechsten Vakuumleitung 85 eine beispielsweise als Druckmessröhre ausgebildete Druckmesseinrichtung 86 zur Messung insbesondere des Unterdrucks innerhalb der Plasmakammer 17 zugeordnet sein. Schließlich kann vorzugsweise zwischen der ersten Seite 85.1 der sechsten Vakuumleitung 85 und deren Ventileinrichtung 85.3 eine dritte Belüftungsleitung 87 zur Beaufschlagung der Plasmakammer 17 bzw. dem Innenraum der Kavität 4 mit dem Sterilisationsmedium vorgesehen sein, die von der sechsten Vakuumleitung 85 fluiddicht abgezweigt und über eine ihr zugeordnete regel- und/oder steuerbare Ventileinrichtung 87.1 zu- bzw. abschaltbar ausgebildet ist. In anderen Worten kann also über die sechste Vakuumleitung 85 sowohl ein Unterdruck in dem Innenraum der Kavität 4 erzeugt, als auch ein Sterilisationsmedium über die abgezweigte dritte Belüftungsleitung 87 eingeleitet werden.

Dabei können insbesondere die erste, zweite sowie dritte Belüftungsleitung 76, 84, 87 zur Betriebsmittelversorgung mit der in Figur 2 dargestellten Ringleitung 21 des Drehverteilers 20 zusammenwirken, bzw. an dieser angeschlossen sein. Dabei können die der Plasmakammer 17 abgewandten Enden der ersten, zweiten sowie dritten Belüftungsleitung 76, 84, 87 mit einer allen drei Belüftungsleitungen 76, 84, 87 gemeinsamen oder auch jeweils separaten, nicht nähergehend dargestellten, Sterilisationseinrichtung zusammenwirken. Die Sterilisationseinrichtung kann zur Herstellung bzw. Produktion des Sterilisationsmediums ausgebildet sein, oder als Vorlagetank oder als Aufbereitungsstation für das Sterilisationsmedium. Wenigstens die erste, zweite sowie dritte Belüftungsleitung 76, 84, 87 können dabei insbesondere aus einem Material hergestellt bzw. gefertigt sein, das resistent gegen einen chemischen Angriff oder Reaktion mit dem Sterilisationsmedium, insbesondere H2O2 oder O3, ausgebildet ist.

Weiterhin kann eine nur schematisch angedeutete und in die Plasmakammer 17 einmündende Saugleitung 88 vorgesehen sein, um noch restlich vorhandenes Sterilisationsmedium nach erfolgter Beaufschlagung zumindest des Behälterinnenraums 5.1 mit dem Sterilisationsmedium und vor einer erneuten Plasmabehandlung eines weiteren Behälters 5 in der Plasmakammer 17 insbesondere aus der ersten bis dritten sowie der zentralen Prozessgasleitung 80...83 abzusaugen. Dabei kann der Saugleitung 88 eine regel-und/oder steuerbare Ventileinrichtung 88.1 sowie ein Volumenstrommessgerät 88.2 zugeordnet sein. Mithin kann eine Absaugströmung in der Saugleitung 88 über eine nicht näher dargestellte Absaugeinrichtung erzeugt werden. Insbesondere kann die Absaugung über die Saugleitung 88 bei angehobener Behältermündung bzw. abgesenkten Lanzenschlitten 37 erfolgen. Das besondere dieser zusätzlichen Saugleitung 88 und der dazugehörigen Komponenten ist, dass diese für den Beschichtungsprozess nicht erforderlich sind und somit konstruktiv nicht zur Erreichung von Hochvakuum geeignet sein müssen. Da diese nur zum Absaugen von restlichem Sterilisationsmedium dienen, muss bspw. die Saugleitung 88 vorrangig hinsichtlich Korrosionsbeständigkeit ausgelegt sein. Hierzu kann die Saugleitung 88 beispielsweise aus einem Kunststoff (bspw. Teflon), Edelstahl oder einem gegen Korrosion beschichteten sonstigen Material sein. Durch das Vorsehen dieser zusätzlichen Saugleitung 88 wird der Kontakt der zentralen Vakuumeinrichtung 77 und der entsprechenden Zuleitungen und Komponenten mit den zum Teil sehr korrosiven Sterilisationsmedien gemindert oder vorständig vermieden. Weiterhin können durch die Entkopplung der Prozessschritte die Prozesszeiten für die zentrale Vakuumeinrichtung 77 auf quasi unvermindertem Niveau gehalten werden.

Ein typischer Behandlungsvorgang wird im Folgenden am Beispiel eines Beschichtungsvorgangs erläutert und derart durchgeführt, dass zunächst der Behälter 5 unter Verwendung des Eingaberades zum Plasmarad 2 transportiert wird und dass in einem hochgeschobenen Zustand der hülsenartigen Kammerwandung 18 das Einsetzen des Behälters 5 in die Plasmastation 3 erfolgt. Nach einem Abschluss des Einsetzvorgangs wird die Kammerwandung 18 in ihre abgedichtete Positionierung abgesenkt und zunächst gleichzeitig eine Evakuierung sowohl der Kavität 4 als auch des Behälterinnenraums 5.1 des Behälters 5 durchgeführt.

Nach einer ausreichenden Evakuierung des Innenraums der Kavität 4 wird die Gaslanze 36 in den Behälterinnenraum 5.1 des Behälters 5 eingefahren und durch eine Verschiebung des Dichtelement 28 eine Abdichtung des Behälterinnenraums 5.1 gegenüber dem Innenraum der Kavität 4 durchgeführt. Ebenfalls möglich kann die Gaslanze 36 bereits synchron zur beginnenden Evakuierung des Innenraums der Kavität 4 in den Behälter 5 hinein verfahren werden. Anschließend kann der Druck im Behälterinnenraum 5.1 noch weitergehend abgesenkt werden. Darüber hinaus ist auch daran gedacht, die Positionierbewegung der Gaslanze 36 wenigstens teilweise bereits parallel zur Positionierung der Kammerwandung 18 durchzuführen. Nach Erreichen eines ausreichend tiefen Unterdrucks wird Prozessgas in den Behälterinnenraum 5.1 des Behälters 5 eingeleitet und mit Hilfe des Mikrowellengenerators 19 das Plasma gezündet. Insbesondere kann vorgesehen sein, dass mit Hilfe des Plasmas sowohl ein Haftvermittler auf eine innere Oberfläche des Behälters 5 als auch die eigentliche Barriereschicht aus Siliziumoxiden abgeschieden wird.

Nach einem Abschluss des Beschichtungsvorgangs, also der Plasmabehandlung, wird die Gaslanze 36 wieder aus dem Behälterinnenraum 5.1 entfernt, d. h. abgesenkt, und synchron oder dem Absenken der Gaslanze 36 zeitlich vorgelagert wenigstens der Behälterinnenraum 5.1 des Behälters 5 sowie ggfs. die Plasmakammer 17 wenigstens teilweise mit einem Sterilisationsmedium belüftet, d.h. mit dem Sterilisationsmedium beaufschlagt. Über die Belüftung der Behälterinnenraum 5.1 des Behälters 5 sowie ggfs. die Plasmakammer 17 kann also das Sterilisationsmedium eingeleitet werden. Die Belüftung wenigstens des Behälterinnenraums 5.1 mit dem Sterilisationsmedium findet dabei insbesondere noch an einer Plasmastation 3 des Plasmamoduls 1 statt, besonders bevorzugt, wenn sich diese Plasmastation 3 im Bereich des Ausgaberads 12 befindet, ohne dieses jedoch erreicht zu haben.

Erfindungsgemäß eignet sich dabei als Sterilisationsmedium ein Wasserstoffperoxyd (H2O2) und/oder Ozon (O3) enthaltendes Sterilisationsmedium, das beispielweise zusammen mit heißer steriler Luft verwendet wird und sich damit an dem kälteren Behälterinnenraum 5.1 beispielweise als H2O2-Kondensationsfilm niederschlägt. Erfindungsgemäß kann das Sterilisationsmedium dabei in einem gas-, dampf- oder nebelförmigen Aggregatszustand vorliegen.

Es kann auch vorgesehen sein, dass das derart in den Behälterinnenraum 5.1 eingebrachte Sterilisationsmedium nachfolgend aktiviert und/oder getrocknet wird, indem beispielweise heißes gas- und/oder dampfförmiges Aktivierungsmedium in den Behälterinnenraum 5.1 derart eingebracht wird, dass durch einen Zerfall von H2O2 freie Sauerstoffradikale entstehen, die zur Sterilisation der Behälter 5 mit vorhanden Keimen und Verunreinigungen reagieren. Beispielsweise kann das Aktivierungsmedium als sterile heiße Luft mit einer Temperatur von 130 °C bis 150 °C ausgebildet sein.

Die Aktivierung und/oder Trocknung des Behälterinnenraums 5.1 kann beispielweise am Ausgaberad 12 erfolgen. Alternativ kann die Aktivierung und/oder Trocknung des jeweiligen Behälters 5 auch beim Transport des mit Sterilisationsmedium beaufschlagten Behälters 5 zur nachfolgenden Behälterbehandlungsmaschine, beispielweise einem Füller, erfolgen. Hierbei kann zusätzlich vorgesehen sein, dass zumindest der Bereich des Ausgaberades 12 des Plasmamoduls 1 derart eingehaust ist, dass ein steriles Umfeld sichergestellt ist. Beispielsweise kann die vorgesehene Einhausung mit steriler Luft betrieben werden. Es kann zudem auch die Transportstrecke zur nachfolgenden Behälterbehandlungsmaschinen eingehaust ausgebildet und/oder die mit Sterilisationsmedium beaufschlagten Behälter 5 auf bzw. während des Transports zur nachfolgenden Behälterbehandlungsmaschine aktiviert und/oder getrocknet werden. Derartige sterile Einhausungen sind dem Fachmann bekannt und bedürfen daher keiner nähergehenden Erläuterung.

Eine Positionierung der Kammerwandung 18, des Dichtelements 28 und/oder der Gaslanze 36 kann unter Verwendung unterschiedlicher Antriebsaggregate erfolgen. Grundsätzlich ist die Verwendung pneumatischer Antriebe und/oder elektrischer Antriebe, insbesondere in einer Ausführungsform als Linearmotor, denkbar. Insbesondere ist aber daran gedacht, zur Unterstützung einer exakten Bewegungskoordinierung mit einer Rotation des Plasmarades 2 eine Kurvensteuerung zu realisieren. Die Kurvensteuerung kann beispielsweise derart ausgeführt sein, dass entlang eines Umfangs des Plasmarades 2 Steuerkurven angeordnet sind, entlang derer Kurvenrollen geführt werden. Die Kurvenrollen sind mit den jeweils zu positionierenden Bauelementen gekoppelt.

Zunächst wird nach einem Schließen der Plasmakammer 17 beispielsweise die erste und sechste Ventileinrichtung 71.1 bzw. 85.1 geöffnet und damit über die erste bzw. sechste Vakuumleitung 71 bzw. 85 sowohl der Behälterinnenraum 5.1 als auch der Innenraum der Plasmakammer 17 evakuiert. Dies passiert bei zudem geöffneter Ventileinrichtung 79.1 der Absperrleitung 79. Bevorzugt sind während dessen die Ventileinrichtung 80.1 der zentralen Prozessgasleitung 80 sowie die Ventileinrichtung 88.1 der Saugleitung 88 geschlossen. Insbesondere sind während des Evakuierens des Behälterinnenraums 5.1 sowie der Plasmakammer 17 auch die entsprechenden Ventileinrichtungen 76.1, 84.1 und 87.1 der ersten bis dritten Belüftungsleitung 76, 84, 87 geschossen. Nach einem Schließen der ersten Ventileinrichtung 71.1 kann beispielweise die zweite Ventileinrichtung 72.1 geöffnet werden und damit der Behälterinnenraum 5.1 über die zweite Vakuumleitung 72 auf ein niedrigeres Druckniveau abgesenkt werden. Auch kann der Behälterinnenraum 5.1 und/oder die Plasmakammer 17 noch über die dritte oder vierte Vakuumleitung 73, 74 auf weitergehend niedrigere Unterdruckstufen abgesenkt werden, wenn dies erforderlich ist. Nach Erreichen eines ausreichend tiefen Druckniveaus in dem Behälterinnenraum 5.1 und/oder der Plasmakammer 17 können die entsprechenden Ventileinrichtungen 71.1...75.1 geschlossen werden. Alternativ kann auch vorgesehen sein, dass zur Bereitstellung eines weitergehend ausreichend tiefen Druckniveaus im Behälterinnenraum 5.1 und der Plasmakammer 17 während der nachfolgenden Behandlungsschritte insbesondere die fünfte Ventileinrichtung 75.1 sowie die sechste Ventileinrichtung 85.1 geöffnet bleiben.

Dabei kann gleichzeitig oder zeitlich vorgelagert zu einer Positionierung der Gaslanze 36 innerhalb des Behälterinnenraums 5.1 bereits eine oder mehrere der ersten bis dritten Ventileinrichtungen 81.1...83.1 der ersten bis dritten Prozessgasleitungen 81...83 sowie die Ventileinrichtung 80.1 der zentralen Prozessgasleitung 80 geöffnet werden und ein Prozessgas einer bestimmten Zusammensetzung insbesondere dem Behälterinnenraum 5.1 über die Gaslanze 36 zugeführt werden. Dazu zeitlich vorgelagert oder gleichzeitig wird die Ventileinrichtung 79.1 der Absperrleitung 79 geschlossen.

Nach einer ausreichenden Prozessgaszuführung zündet der Mikrowellengenerator 19 das Plasma im Behälterinnenraum 5.1 des Behälters 5. In diesem Zusammenhang kann vorgesehen sein, dass beispielsweise die Ventileinrichtung 81.1 der ersten Prozessgasleitung 81 zu einem vorgebaren Zeitpunkt schließt, während die Ventileinrichtung 82.1 der zweiten Prozessgasleitung 82 zur Zuführung eines Prozessgases einer zweiten Zusammensetzung geöffnet wird. Zumindest zeitweise kann dabei auch die fünfte Ventileinrichtung 76.1 und/oder die sechste Ventileinrichtung 85.3 geöffnet sein, um einen ausreichend niedrigen Unterdruck insbesondere im Behälterinnenraum 5.1 und/oder der Prozesskammer 17 aufrecht zu halten. Hierbei erweist sich ein Druckniveau von ca. 0,3 mbar als zweckmäßig.

Nach Abschluss der Plasmabehandlung werden die Ventileinrichtungen 81.1 ...83.1 der ersten bis dritten Prozessgasleitung 81 ...83 sowie sämtliche zu diesem Zeitpunkt noch geöffneten Ventileinrichtungen 71.1...75.1, 85.3 der ersten bis sechsten Vakuumleitung 71...75, 85 geschlossen, während zumindest die Ventileinrichtung 84.1 der zweiten Belüftungsleitung 84 geöffnet und zumindest der Behälterinnenraum 5.1 des Behälters 5 nach der Plasmabehandlung an der Plasmastation 3 wenigstens teilweise mit einem Sterilisationsmedium belüftet, d.h. beaufschlagt, wird. Bevorzugt wird dabei das Sterilisationsmedium über die Gaslanze 36 in den Behälterinnenraum 5.1 eingebracht. Synchron dazu kann die Gaslanze 36 aus dem Behälterinnenraum 5.1 abgesenkt und/oder die Ventileinrichtung 76.1 der zweiten Belüftungsleitung 76 geöffnet werden, um damit zumindest den Behälterinnenraum 5.1 des Behälters 5 nach der Plasmabehandlung an der Plasmastation 3 wenigstens teilweise mit einem Sterilisationsmedium zu belüften, d.h. zu beaufschlagen. Weiterhin kann damit auch eine Belüftung bzw. Beaufschlagung der Plasmakammer 17 bzw. der Behälteraußenwandung des Behälters 5 mit Sterilisationsmedium erfolgen. Weiterhin kann die Belüftung der Plasmakammer 17 bzw. der Behälteraußenwandung des Behälters 5 mit Sterilisationsmedium auch erfolgen, indem nachfolgend die Ventileinrichtung 87.1 der dritten Belüftungsleitung 87 geöffnet wird.

Nach einer ausreichenden Beaufschlagung bzw. Belüftung des Behälterinnenraums 5.1 und der Plasmakammer 17 mit dem Sterilisationsmedium vorzugsweise bis wenigstens Atmosphärendruck, bzw. Umgebungsdruck werden die geöffneten Ventileinrichtungen 76.1, 84.1, 87.1 der ersten bis dritten Belüftungsleitung 76, 84, 87 geschlossen. Dabei beträgt die Belüftungszeit je Behälter 5 zwischen 0,1 und 0,4 Sekunden, vorzugsweise etwa 0,2 Sekunden. Weiterhin beträgt die Verweilzeit des jeweiligen mit Sterilisationsmedium beaufschlagten Behälter 5 nach dem Belüften noch etwa 2,5 Sekunden an dem Plasmamodul 1, bis der entsprechende Behälter 5 über das Ausgaberad 12 an eine weitere Transportstrecke übergeben wird.

Nachfolgend kann eine Spülung und/oder Absaugung des restlichen Sterilisationsmediums über beispielweise die vierte und/oder fünfte Vakuumleitung 74, 75 und/oder die Saugleitung 88 erfolgen, um das nach der Belüftung noch in der ersten bis dritten sowie zentralen Prozessgasleitung 80...83 und der Plasmakammer 17 vorhandene Sterilisationsmedium zu beseitigen. Weiterhin kann auch vorgesehen sein, dass über die Absperrventileinrichtung 79 nach erfolgtem Belüften zumindest des Behälterinnenraums 5.1 ein Spülmedium in die Versorgungskreise eingeleitet wird, und dadurch die mit Sterilisationsmedium kontaminierten Versorgungskreise gereinigt werden.

Nach Erreichen des Umgebungsdruckes innerhalb der Kavität 4 wird die Kammerwandung 18 wieder angehoben. Nachfolgend erfolgt eine Entnahme bzw. Übergabe des beschichteten und mit Sterilisationsmedium beaufschlagten Behälters 5 an das Ausgaberad 12.

### Bezugszeichenliste

- 1: Plasmamodul
- 2: Plasmarad
- 3: Plasmastation
- 4: Kavität
- 5: Behälter
- 5.1: Behälterinnenraum
- 6: Eingabe
- 7: Vereinzelungsrad
- 8: Übergaberad
- 9: Tragarm
- 10: Sockel
- 11: Eingaberad
- 12: Ausgaberad
- 13: Ausgabestrecke
- 14: Tragring
- 15: Maschinensockel
- 16: Stationsrahmen
- 17: Plasmakammer
- 18: Kammerwandung
- 19: Mikrowellengenerator
- 20: Drehverteiler
- 21: Ringleitung
- 23: Führungsstange
- 24: Schlitten
- 25: Umlenkung
- 26: Adapter
- 27: Kopplungskanal
- 28: Halteelement
- 29: Kammerboden
- 30: Kammersockel
- 31: Kammerdeckel
- 32: Flansch
- 33: Dichtung
- 34: Innenflansch
- 35: Dichtung
- 36: Gaslanze
- 37: Lanzenschlitten
- 38: Prozessgaskanal
- 39: Gasanschluss
- 70: Vakuumkanal
- 70.1: erste Seite
- 70.2: zweite Seite
- 71: erste Vakuumleitung
- 71.1: Ventileinrichtung
- 72: zweite Vakuumleitung
- 72.1: Ventileinrichtung
- 73: dritte Vakuumleitung
- 73.1: Ventileinrichtung
- 74: vierte Vakuumleitung
- 74.1: Ventileinrichtung
- 75: fünfte Vakuumleitung
- 75.1: Ventileinrichtung
- 76: erste Belüftungsleitung
- 76.1: Ventileinrichtung
- 77: Vakuumeinrichtung
- 78: Druckmesseinrichtung
- 78.1: Ventileinrichtung
- 79: Absperrventileinrichtung
- 80: zentrale Prozessgasleitung
- 80.1: Ventileinrichtung
- 81: erste Prozessgasleitung
- 81.1: Ventileinrichtung
- 82: zweite Prozessgasleitung
- 82.2: Ventileinrichtung
- 83: dritte Prozessgasleitung
- 83.1: Ventileinrichtung
- 84: zweite Belüftungsleitung
- 84.1: Ventileinrichtung
- 85: sechste Vakuumleitung
- 85.1: erste Seite
- 85.2: zweite Seite
- 85.3: Ventileinrichtung
- 86: Druckmesseinrichtung
- 87: Belüftungsleitung
- 87.1: Ventileinrichtung
- 88: Saugleitung
- 88.1: Ventileinrichtung
- 88.2: Volumenstrommessgerät

## Patentansprüche

1. Verfahren zur Plasmabehandlung von Behältern (5), bei welchem
- mindestens ein Behälter (5) mit einem Behälterinnenraum (5.1) in eine Plasmakammer (17) einer Plasmastation (3) eingesetzt und positioniert wird,
- die Plasmakammer (17) und der mindestens eine Behälterinnenraum (5.1 ) zumindest teilweise evakuiert werden,
- zumindest der eine Behälterinnenraum (5.1) des Behälters (5) innerhalb der zumindest teilweise evakuierten Plasmakammer (17) mit einer Innenbeschichtung mittels Plasmabehandlung versehen wird, und
- nach der Plasmabehandlung ein Belüftungsschritt folgt, bei welchem sowohl die Plasmakammer (17) als auch der mindestens eine Behälterinnenraum (5.1) des Behälters (5) zumindest teilweise belüftet werden,
**dadurch gekennzeichnet, dass** zumindest der Behälterinnenraum (5.1) des wenigstens einen Behälters (5) nach der Plasmabehandlung und vor dem Befüllen des Behälters (5) in der Plasmakammer (17) mit einem gas-, dampf- oder nebelförmigen Sterilisationsmedium Sterilisationsmedium derart beaufschlagt wird, dass bei dem Belüftungsschritt zumindest der Behälterinnenraum (5.1 ) zumindest teilweise mit dem gas-, dampf- oder nebelförmigen Sterilisationsmedium belüftet wird, wobei die Beaufschlagung wenigstens des Behälterinnenraums (5.1) des Behälters (5) mit einem Wasserstoffperoxid und/oder Ozon enthaltenden Sterilisationsmedium erfolgt.

2. Verfahren nach Anspruch 1, **dadurch**
**gekennzeichnet, dass** der Behälterinnenraum (5.1) des Behälters (5) wenigstens bis auf Atmosphärendruck mit dem Sterilisationsmedium beaufschlagt wird.

3. Verfahren nach einem der vorhergehenden Ansprüche 1 bis 2, **dadurch**
**gekennzeichnet, dass** neben dem Behälterinnenraum (5.1 ) des Behälters (5) auch die Plasmakammer (17) wenigstens teilweise mit dem Sterilisationsmedium belüftet wird.

4. Verfahren nach einem der vorhergehenden Ansprüche 1 bis 3, **dadurch**
**gekennzeichnet, dass** eine nachfolgende Aktivierung und/oder Trocknung des Sterilisationsmediums durch ein Aktivierungsmedium, ausgebildet als sterile heiße Luft mit einer Temperatur von 130 °C bis 150 °C, erfolgt.

5. Verfahren nach Anspruch 4, **dadurch gekennzeichnet, dass** die Aktivierung des jeweiligen Behälters (5) während seines Transports zu einer einem Plasmamodul (1) nachgelagerten Behälterbehandlungsmaschine erfolgt.

6. Verfahren nach einem der Ansprüche 4 bis 5, **dadurch gekennzeichnet, dass** die Trocknung des jeweiligen Behälters (5) während seines Transports zu einer einem Plasmamodul (1) nachgelagerten Behälterbehandlungsmaschine erfolgt.

7. Verfahren nach einem der vorhergehenden Ansprüche 1 bis 6, **dadurch**
**gekennzeichnet, dass** vor einer erneuten Plasmabehandlung wenigstens eine erste bis dritte Prozessgasleitung (81 ...83) sowie eine zentrale Prozessgasleitung (80) abgesaugt und/oder gespült wird.

8. Verfahren nach einem der vorhergehenden Ansprüche 1 bis 7 , **dadurch gekennzeichnet, dass** die Belüftung mittels Sterilisationsmedium wenigstens über eine in die zentrale Prozessgasleitung (80) fluiddicht einmündende zweite Belüftungsleitung (84) erfolgt, die über eine Ventileinrichtung (84.1) gesteuert und/oder geregelt zu- und/oder abschaltbar ausgebildet ist, wobei die Belüftung mittels Sterilisationsmedium über eine in eine zweite Seite (70.2) eines Vakuumkanals (70) fluiddicht einmündende weitere erste Belüftungsleitung (76) erfolgt, die über eine Ventileinrichtung (76.1) gesteuert und/oder geregelt zu- und/oder abschaltbar ausgebildet ist, und/oder wobei die Belüftung mittels Sterilisationsmedium über eine nochmals weitere dritte fluiddicht in die Plasmakammer (17) einmündende Belüftungsleitung (87) erfolgt, die über eine Ventileinrichtung (87.1) gesteuert und/oder geregelt zu- und/oder abschaltbar ausgebildet ist.

9. Verfahren nach einem der vorhergehenden Ansprüche 1 bis 8, **dadurch gekennzeichnet, dass** in einer Fluidverbindung zwischen der Vakuumvorrichtung (77) und der Kavität (4) eine zusätzliche Absperrventileinrichtung (79) vorgesehen ist, welche mindestens zeitweise während des Belüftens geschlossen wird, um eine Kontamination mindestens einer der Vakuumleitungen (71 bis 75), einer Gruppe von Vakuumleitungen (71 bis 75) oder Teilabschnitten einer oder mehrerer Vakuumleitungen (71 bis 75) mit Sterilisationsmedium zu verhindern.

10. Vorrichtung zur Plasmabehandlung von Behältern (5), mit einem Verfahren nach Ansprüchen 1 - 9 umfassend wenigstens eine an einem Plasmarad (2) angeordnete Plasmastation (3) mit jeweils wenigstens einer Plasmakammer (17), in welcher mindestens ein Behälter (5) mit einem Behälterinnenraum (5.1) einsetzbar und positionierbar ist, wobei die jeweilige Plasmakammer (17) zumindest teilweise evakuierbar ausgebildet ist, wobei die Plasmastation (3) dazu eingerichtet ist, den zumindest einen Behälterinnenraum (5.1) des Behälters (5) innerhalb der zumindest teilweise evakuierten Plasmakammer (17) mit einer Innenbeschichtung mittels Plasmabehandlung auszubilden und wobei die Plasmastation (3) wenigstens eine Belüftungsleitung (76, 84, 87) zum zumindest teilweisen Belüften sowohl der Plasmakammer (17) als auch des zumindest einen Behälterinnenraumes (5.1) nach der Plasmabehandlung aufweist, **dadurch gekennzeichnet, dass** wenigstens eine Belüftungsleitung (76, 84, 87) zum Beaufschlagen zumindest des Behälterinnenraumes (5.1) des wenigstens einen Behälters (5) mit einem gas-, dampf- oder nebelförmigen Sterilisationsmedium vorgesehen ist, wobei die Beaufschlagung wenigstens des Behälterinnenraums (5.1) des Behälters (5) mit einem Wasserstoffperoxid und/oder Ozon enthaltenden Sterilisationsmedium erfolgt, und wobei die wenigstens eine Belüftungsleitung (76, 84, 87) mit einer Versorgungseinheit für das Wasserstoffperoxid und/oder Ozon enthaltende gas-, dampf- oder nebelförmige Sterilisationsmedium verbunden ist.

11. Vorrichtung nach Anspruch 10, **dadurch gekennzeichnet, dass** die wenigstens eine Belüftungsleitung (76, 84, 87) mit ihrem der Plasmakammer (17) abgewandten Ende mit einer Sterilisationseinrichtung verbunden ist.

12. Vorrichtung nach Anspruch 10 oder 11, **dadurch gekennzeichnet, dass** wenigstens eine zweite Belüftungsleitung (84) zum Belüften mittels Sterilisationsmedium vorgesehen ist, die fluiddicht in eine zentrale Prozessgasleitung (80) einmündet, und die über die über eine Ventileinrichtung (84.1) gesteuert und/oder geregelt zu- und/oder abschaltbar ausgebildet ist, wobei eine erste Belüftungsleitung (76) zum Belüften mittels Sterilisationsmedium vorgesehen ist, die fluiddicht in eine zweite Seite (70.2) eines Vakuumkanals (70) einmündet, und die über eine Ventileinrichtung (76.1) gesteuert und/oder geregelt zu- und/oder abschaltbar ausgebildet ist.

13. Vorrichtung nach Anspruch 12, **dadurch gekennzeichnet, dass** eine dritte Belüftungsleitung (76) zum Belüften mittels Sterilisationsmedium vorgesehen ist, die fluiddicht in die Plasmakammer (17) einmündet, und die über eine Ventileinrichtung (87.1) gesteuert und/oder geregelt zu- und/oder abschaltbar ausgebildet ist.

14. Vorrichtung nach einem der vorhergehenden Ansprüche 10 bis 13, **dadurch gekennzeichnet, dass** zur Absaugung des Sterilisationsmediums eine in die Plasmakammer (17) einmündende Saugleitung (88) vorgesehen ist.

15. Vorrichtung nach einem der vorhergehenden Ansprüche 10 bis 14, **dadurch gekennzeichnet, dass** im Bereich eines Ausgaberades (12) eine Aktivierungs- und/oder Trocknungseinrichtung vorgesehen ist zur Aktivierung und/oder Trocknung des Sterilisationsmediums durch ein Aktivierungsmedium, ausgebildet als sterile heiße Luft mit einer Temperatur von 130 °C bis 150 °C.

## Claims

1. A method for the plasma treatment of containers (5) in which
- at least one container (5) with a container interior (5.1) is inserted and positioned into a plasma chamber (17) of a plasma station (3),
- the plasma chamber (17) and the at least one container interior (5.1) are at least partially evacuated,
- at least one container interior (5.1) of the container (5) within the at least partially evacuated plasma chamber (17) is provided with an inner coating by means of plasma treatment, and
- after plasma treatment, a aeration step follows, in which both the plasma chamber (17) as well as the at least one container interior (5.1) of the container (5) are at least partially aerated,
**characterized in that** at least the container interior (5.1) of at least one container (5) is impinged with a gaseous, vaporous or misty sterilization medium in the plasma chamber (17) after plasma treatment and before filling of the container (5) in such a way that at least the container interior (5.1) is at least partially aerated with the gaseous, vaporous or misty sterilization medium during the aeration step, wherein the impingement of at least the container interior (5.1) of the container (5) with a sterilization medium containing hydrogen peroxide and/or ozone occurs.

2. The method according to Claim 1, **characterized in that** the container interior (5.1) of the container (5) is impinged with the sterilization medium at least up to atmospheric pressure.

3. The method according to any one of the preceding Claims 1 to 2, **characterized in that**, in addition to the container interior (5.1) of the container (5), the plasma chamber (17) is also at least partially aerated with the sterilization medium.

4. The method according to any one of the preceding Claims 1 to 3, **characterized in that** a subsequent activation and/or drying of the sterilization medium is carried out by an activation medium formed as sterile hot air at a temperature of 130 °C to 150 °C.

5. The method according to Claim 4, **characterized in that** the activation of the respective container (5) occurs during its transport to a container treatment machine downstream of a plasma module (1).

6. The method according to any one of the Claims 4 to 5, **characterized in that** the drying of the respective container (5) occurs during its transport to a container treatment machine downstream of a plasma module (1).

7. The method according to any one of the preceding Claims 1 to 6, **characterized in that** at least one first to third process gas line (81 ... 83) as well as a central process gas pipeline (80) is extracted and/or flushed.

8. The method according to any one of the preceding Claims 1 to 7, **characterized in that** aeration by means of sterilization medium occurs at least via one second aeration line (84) flowing in a fluid-tight manner into the central process gas line (80), which is designed to be to capable of being engaged and/or disengaged in a controlled and/or regulated manner by means of a valve device (84.1), wherein aeration by means of sterilization medium occurs via further first aeration line (76) flowing in a fluid-tight manner into a second side (70.2) of a vacuum channel (70) which is designed to be capable of being engaged and/or disengaged in a controlled and/or regulated manner by means of a valve device (76.1) and/or wherein aeration is carried out by means of a sterilization medium via a further third aeration line (87) flowing in a fluid-tight manner into the plasma chamber (17), which is designed to be capable of being engaged and/or disengaged in a controlled and/or regulated manner via a valve device (87.1).

9. The method according to any one of the preceding Claims 1 to 8, **characterized in that** an additional shut-off-valve device (79) is provided in a fluid connection between the vacuum device (77) and the cavity (4), which is closed at least temporarily during aeration, in order to prevent contamination of at least one of the vacuum lines (71 to 75), a group of vacuum lines (71 to 75) or parts of one or a plurality of vacuum lines (71 to 75) with sterilization medium.

10. A device for the plasma treatment of containers (5) with a method according to Claims 1 - 9 comprising at least one plasma station (3) arranged on a plasma wheel (2) each with at least one plasma chamber (17), in which at least one container (5) with a container interior (5.1) can be inserted and positioned, wherein the respective plasma chamber (17) is at least partially designed to be evacuable, wherein the plasma station (3) is configured for the container interior (5.1) of the container (5) within the plasma chamber (17), which has been at least partially evacuated, to be designed with an inner coating by means of plasma treatment, and wherein the plasma station (3) comprises at least one aeration line (76, 84, 87) for at least partial aeration of both the plasma chamber (17) as well as the at least one container interior (5.1) after plasma treatment, **characterized in that** at least one aeration line (76, 84, 87) is provided for impinging at least one container interior (5.1) of at least one container (5) with a gaseous, vaporous or misty sterilization medium, wherein the impingement of at least the container interior (5.1) of the container (5) occurs using a sterilization medium containing hydrogen peroxide and/or ozone, and wherein at least one aeration line (76, 84, 87) is connected to a supply unit for the gaseous, vaporous or misty sterilization medium containing hydrogen peroxide and/or ozone.

11. The device according to Claim 10, **characterized in that** at least one aeration line (76, 84, 87) is connected to a sterilization device with its end facing away from the plasma chamber (17).

12. The device according to Claim 10 or 11, **characterized in that** at least one second aeration line (84) is provided for aeration by means of sterilization medium, which flows in a fluid-tight manner into a central process gas line (80) and which is designed to be capable of being engaged and/or disengaged in a controlled and/or regulated manner by means of a valve device (84.1), wherein a first aeration line (76) is provided for aeration by means of sterilization medium which flows in fluid-tight manner into a second side (70.2) of a vacuum channel (70), and which is designed to be capable of being engaged and/or disengaged in a controlled and/or regulated manner by means of a valve device (76.1).

13. The device according to Claim 12, **characterized in that** a third aeration line (76) is provided for aeration by means of sterilization medium, which flows in a fluid-tight manner into the plasma chamber (17) and which is designed to be capable of engaged and/or disengaged in a regulated and/or controlled manner by means of a valve device (87.1).

14. The device according to any one of the preceding Claims 10 to 13, **characterized in that** a suction line (88) is provided for the extraction of the sterilization medium flowing into the plasma chamber (17).

15. The device according to any one of the preceding Claims 10 to 14, **characterized in that** an activation and/or drying device is provided in the region of an output wheel (12) for the activation and/or drying of the sterilization medium by an activation medium formed as sterile hot air at a temperature of 130 °C to 150 °C.

## Revendications

1. Procédé de traitement au plasma de récipients (5) dans le cadre duquel
- au moins un récipient (5) avec un espace intérieur (5.1) peut être introduit et positionné dans une chambre à plasma (17) d'une station de plasma (3),
- la chambre à plasma (17) et l'espace intérieur (5.1) du (des) récipient(s) sont en partie au moins évacués,
- au moins l'espace intérieur (5.1) du (des) récipient(s) est revêtu d'un revêtement par traitement au plasma à l'intérieur de la chambre à plasma (17) évacuée au moins partiellement, et
- une étape de ventilation lors de laquelle la chambre à plasma (17) aussi bien que l'espace intérieur (5.1) du (des) récipient(s) (5) sont en partie au moins aérés suit le traitement au plasma.
**caractérisé en ce que,** après le traitement au plasma et avant le remplissage du récipient (5), au moins l'espace intérieur (5.1) du (des) récipient(s) (5) est soumis dans la chambre à plasma (17) à l'action d'un agent de stérilisation gazeux ou sous forme de vapeur ou de brume de façon à ce que, pendant l'étape de ventilation, la ventilation d'au moins l'espace intérieur (5.1) du récipient est, en partie au moins, effectuée avec l'agent de stérilisation gazeux ou sous forme de vapeur ou de brume, l'agent de stérilisation à l'action duquel l'espace intérieur (5.1) du récipient (5) est soumis contenant du peroxyde d'hydrogène et/ou de l'ozone.

2. Procédé selon la revendication 1, **caractérisé en ce que** l'espace intérieur (5.1) du récipient (5) est soumis à l'action de l'agent de stérilisation au moins jusqu'à la pression atmosphérique.

3. Procédé selon l'une des revendications précédentes 1 à 2, **caractérisé en ce que,** outre l'espace intérieur (5.1) du récipient (5), la chambre à plasma (17) est également ventilée au moins partiellement avec l'agent de stérilisation.

4. Procédé selon l'une des revendications précédentes 1 à 3, **caractérisé en ce que,** dans une étape suivante est effectué une activation et/ou un séchage de l'agent de stérilisation par un agent d'activation sous forme d'air chaud stérile d'une température de 130 °C à 150 °C.

5. Procédé selon la revendication 4, **caractérisé en ce que** l'activation de chaque récipient (5) a lieu pendant son transport vers une machine de traitement de récipients placée en aval d'un module à plasma (1).

6. Procédé selon l'une des revendications 4 à 5, **caractérisé en ce que** le séchage de chaque récipient (5) a lieu pendant son transport vers une machine de traitement de récipients placée en aval d'un module à plasma (1).

7. Procédé selon l'une des revendications précédentes 1 à 6, **caractérisé en ce que,** avant un nouveau traitement au plasma, au moins une première à troisième conduite de gaz de procédé 81 ...83) ainsi qu'une conduite de gaz de procédé centrale (80) sont vidées ou et/ou rincées..

8. Procédé selon l'une des revendications précédentes 1 à 7, **caractérisé en ce que** la ventilation au moyen d'un agent de stérilisation a lieu au moins par une seconde conduite de ventilation (84), laquelle débouche de façon étanche dans la conduite de gaz de procédé centrale (80) et est contrôlée et/ou réglée par un dispositif de valve (84.1) de façon à pouvoir être activée et désactivée, la ventilation au moyen d'un agent de stérilisation ayant lieu par une autre première conduite de ventilation (76) laquelle débouche de façon étanche dans un second côté (70.2) d'un canal à vide (70) et est contrôlée et/ou réglée par un dispositif de valve (76.1) de façon à pouvoir être activée et désactivée, et/ou la ventilation au moyen d'un agent de stérilisation ayant lieu par une troisième conduite de ventilation (87) laquelle débouche de façon étanche dans la chambre à plasma (17) et est contrôlée et/ou réglée par un dispositif de valve (87.1) de façon à pouvoir être activée et désactivée.

9. Procédé selon l'une des revendications précédentes 1 à 8, **caractérisé en ce que** dans la conduite à fluide entre le dispositif à vide (77) et la cavité (4) est prévu un dispositif supplémentaire à soupape d'arrêt (79) lequel est fermé temporairement au moins pendant la ventilation pour empêcher une contamination par un agent de stérilisation d'au moins l'une des conduites à vide (71 à 75), d'un groupe de conduites à vide (71 à 75) ou de sections d'une ou de plusieurs conduites à vide (71 à 75).

10. Dispositif de traitement au plasma de récipients (5) par un procédé selon les revendications 1 à 9 comprenant au moins une station de plasma (3) agencée sur une roue (2) de traitement au plasma et comportant respectivement au moins une chambre à plasma (17) dans laquelle peut être introduit et positionné au moins un récipient (5) avec un espace intérieur (5.1), chaque chambre à plasma (17) étant conçue pour pouvoir, en partie au moins, être évacuée, la station de plasma (3) étant conçue pour revêtir l'espace intérieur (5.1) du récipient (5) d'un revêtement par traitement au plasma à l'intérieur de la chambre à plasma (17) évacuée en partie au moins, et la station de plasma (3) présentant au moins une conduite de ventilation (76, 84, 87) pour ventiler au moins partiellement aussi bien la chambre à plasma (17) que l'espace intérieur (5.1) du récipient après le traitement au plasma, **caractérisé en ce qu'**au moins une conduite de ventilation (76, 84, 87) est prévue pour soumettre l'espace intérieur (5.1) du (des) récipient(s) (5) à l'action d'un agent de stérilisation gazeux ou sous forme de vapeur ou de brume, l'agent de stérilisation à l'action duquel l'espace intérieur (5.1) du récipient (5) est soumis contenant du peroxyde d'hydrogène et/ou de l'ozone et la (les) conduite(s) de ventilation (76, 84, 87) étant reliée(s) à une unité d'alimentation fournissant l'agent de stérilisation gazeux ou sous forme de vapeur ou de brume contenant du peroxyde d'hydrogène et/ou de l'ozone.

11. Dispositif selon la revendication 10, **caractérisé en ce que** l'extrémité opposée à la chambre à plasma (17) de la (des) conduite(s) de ventilation (76, 84, 87) est (sont) relié(s) à un dispositif de stérilisation.

12. Dispositif selon la revendication 10 ou 11, **caractérisé en ce qu'**au moins une seconde conduite de ventilation (84) est prévue pour la ventilation au moyen d'un agent de stérilisation, laquelle débouche de façon étanche dans une conduite de gaz de procédé centrale (80) et est contrôlée et/ou réglée par un dispositif de valve (84.1) de façon à pouvoir être activée et désactivée, une première conduite de ventilation (76) étant prévue pour la ventilation au moyen d'un agent de stérilisation, laquelle débouche de façon étanche dans un second côté (70.2) d'un canal à vide (70) et est contrôlée et/ou réglée par un dispositif de valve (76.1) de façon à pouvoir être activée et désactivée.

13. Dispositif selon la revendication 12, **caractérisé en ce qu'**une troisième conduite de ventilation (76) est prévue pour la ventilation au moyen d'un agent de stérilisation, laquelle débouche de façon étanche dans la chambre à plasma (17) et est contrôlée et/ou réglée par un dispositif de valve (87.1) de façon à pouvoir être activée et désactivée.

14. Dispositif selon l'une des revendications précédentes 10 à 13, **caractérisé en ce qu'**une conduite d'aspiration (88) débouchant dans la chambre à plasma (17) est prévue pour aspirer l'agent de stérilisation.

15. Dispositif selon l'une des revendications précédentes 10 à 14, **caractérisé en ce qu'**au niveau de la zone d'une roue de sortie (12) un dispositif d'activation et/ou de séchage est prévu pour l'activation et/ou le séchage de l'agent de stérilisation par un agent d'activation sous forme d'air chaud stérile d'une température de 130 °C à 150 °C.
